# EUROPEAN PATENT APPLICATION

(11) **EP 4 085 904 A1**
(43) Date of publication of application: **09.11.2022**
(21) Application number: 20908952.3
(22) Date of filing: 30.12.2020
(51) Int. Cl.: A61K 9/51

(54) **METHOD FOR PRODUCING NANOPARTICLES COMPRISING LOW-MOLECULAR-WEIGHT AMPHIPHILIC BLOCK COPOLYMER**

(30) Priority: 31.12.2019 KR 20190179161
(71) Applicant: Samyang Holdings Corporation, Seoul 03129 (KR)
(72) Inventor: LEE, Jae Young, Yongin-si Gyeonggi-do 16838 (KR); KIM, Bong Oh, Daejeon 35212 (KR); KIM, Dong Shik, Suwon-si Gyeonggi-do 16349 (KR); LEE, Sa Won, Seongnam-si Gyeonggi-do 13554 (KR)
(74) Representative: Schrell, Andreas
(86) International application number: PCT/KR2020/019374
(87) International publication number: WO 2021/137610

(57) **Abstract**

The present invention relates to a method for producing nanoparticles comprising a low-molecular-weight amphiphilic block copolymer and, more particularly, to a method for producing drug-containing nanoparticles by means of an amphiphilic block copolymer having a predetermined low molecular weight, thereby allowing an increased yield of nanoparticles having superior stability to be produced.

## Description

### TECHNICAL FIELD

The present invention relates to a method for preparing nanoparticle comprising amphiphilic block polymer with low molecular weight, and more specifically, a method for preparing drug-containing nanoparticle by using amphiphilic block polymer with a specific level of low molecular weight, and thereby providing nanoparticle having good stability with increased yield.

### BACKGROUND ART

In order to achieve the desired therapeutic effect of bioactive agent, an appropriate amount of the administered drug must be delivered into the target cell in the body. For this, submicronic particulate drug delivery systems using biodegradable polymer have been researched, and as representatives, nanoparticle system and polymeric micelle system using biodegradable polymer have been reported as technologies that alter the distribution of intravenously administered drug in the body to alleviate side effects and improve efficacy. It has been reported that such drug delivery systems can control the release of drug to target organ, tissue, or cell, have excellent biocompatibility, and improve solubilization capacity of poorly soluble drug and in vivo bioavailability of drug.

The polymer mainly used at present in preparation of polymeric nanoparticle or polymeric micelle is double block amphiphilic block copolymer (mPEG-PLA) consisting of hydrophilic block such as monomethoxypolyethylene glycol (mPEG) and hydrophobic block such as polylactic acid (PLA), and since this polymer has a property of hydrolysis in aqueous solution, it is provided in freeze-dried powder or cake as the final product form, and accordingly, in order to use this polymer as a drug carrier, it should be freeze-dried and stored in solid state, and redissolved in distilled water immediately before use. By the way, such polymers have the disadvantage that it takes a long time to reconstitute when the molecular weight increases.

On the other hand, if the molecular weight decreases to reduce the reconstitution time, there are risks of significantly lowering the quality of the product such as precipitate generation during the preparation process, decrease of nanoparticle stability resulting in precipitation during the storage or transfer, or difficulty in achieving sufficient bioavailability when administered to a patient.

Therefore, there is a need to develop a nanoparticle drug comprising a low molecular weight polymer capable of reducing the reconstitution time without affecting the stability of the nanoparticle.

### CONTENTS OF THE INVENTION

### PROBLEMS TO BE SOLVED

The purpose of the present invention is to provide a method capable of preparing drug-containing nanoparticle that can be reconstituted in reduced time while maintaining stability with high yield, and nanoparticle as such.

### TECHNICAL MEANS

In order to achieve the above purpose, the present invention provides a method for preparing nanoparticle comprising: 1) a step of mixing effective ingredient, amphiphilic block copolymer with a number average molecular weight of less than 3,800, and C₁ to C₅ alcohol; (2) a step of removing C₁ to C₅ alcohol from the resulting mixture of step (1) to lower the amount of C₁ to C₅ alcohol in the mixture to less than 2.5 w/v %; and (3) a step of adding aqueous medium to the resulting mixture of step (2) to form micelle.

In an embodiment, the removal of C₁ to C₅ alcohol can be conducted at a temperature higher than 30 °C, and more concretely, it can be conducted at a temperature higher than 30 °C and lower than 50 °C for a time less than 2 hours.

In an embodiment, the formation of micelle can be conducted at a temperature lower than 35 °C, and more concretely, it can be conducted at a temperature higher than 10 °C and lower than 35 °C.

In an embodiment, the step of forming micelle can comprise a procedure of filtering the formed micelle.

In an embodiment, the preparation method can further comprise a step of freeze-drying the micelle formed in step (3).

In an embodiment, the formed micelle can be maintained at 0 °C to 15 °C before the freeze-drying is conducted.

In an embodiment, the formed micelle can be freeze-dried within 3 hours.

In an embodiment, the amphiphilic block copolymer can comprise hydrophilic block (A) selected from the group consisting of polyethylene glycol or derivative thereof, polyvinyl pyrrolidone, polyvinyl alcohol, polyacrylamide, and combinations thereof; and hydrophobic block (B) selected from the group consisting of polylactide, polyglycolide, poly(lactic-glycolide), polymandelic acid, polycaprolactone, polydioxan-2-one, polyamino acid, polyorthoester, polyanhydride, polycarbonate and combinations thereof.

In an embodiment, the effective ingredient can be poorly water-soluble drug.

Another aspect of the present invention provides nanoparticle comprising effective ingredient; amphiphilic block copolymer with a number average molecular weight of less than 3,800; and C₁ to C₅ alcohol; wherein the amount of the C₁ to C₅ alcohol is 0 to 2.5 w/v %.

In an embodiment, the nanoparticle can be that prepared by the preparation method according to an embodiment of the present invention.

### EFFECT OF THE INVENTION

According to the present invention, the phenomenon of encapsulated drug precipitation during the nanoparticle preparation process is significantly reduced, and thus it is possible to prepare drug-containing nanoparticle having increased stability and remarkably reduced reconstitution time with high yield.

### PREFERRED MODE FOR CARRYING OUT THE INVENTION

The present invention will be explained in detail below.

In the present invention, the term "nanoparticle" can be used for the same meaning as micelle or micelle composition. In an embodiment, the "nanoparticle" can have a sub-micron size (for example, diameter), i.e., less than 1 micrometer (for example, 10 to 900 nm, or 10 to 500 nm).

The "amphiphilic block copolymer" is that can form micelle in aqueous solution, and it can be used for the same meaning as 'micelle polymer.' The amphiphilic block copolymer can be double block copolymer of A-B type consisting of hydrophilic block (A) and hydrophobic block (B), or triple block copolymer of B-A-B type.

In an embodiment, the amount of hydrophilic block in the amphiphilic block copolymer may be 20 to 95 % by weight, and more concretely 40 to 95 % by weight, based on total 100 % by weight of the copolymer. In addition, the amount of hydrophobic block in the amphiphilic block copolymer may be 5 to 80 % by weight, and more concretely 5 to 60 % by weight, based on total 100 % by weight of the copolymer.

In the present invention, the number average molecular weight of the amphiphilic block copolymer is less than 3,800. If the number average molecular weight of the amphiphilic block copolymer is 3,800 or more, the reconstitution time may increase.

In an embodiment, the number average molecular weight of the amphiphilic block copolymer may be 1,500 or more, 2,000 or more, 2,500 or more, 3,000 or more, or 3,300 or more, and it also may be 3,700 or less, 3,600 or less, 3,580 or less, 3,550 or less, 3,500 or less, 3,450 or less, or 3,400 or less.

The hydrophilic block is a polymer having biocompatibility, and concretely, it may comprise one or more selected from the group consisting of polyethylene glycol or derivative thereof, polyvinyl pyrrolidone, polyvinyl alcohol, polyacrylamide, and combinations thereof, and more concretely, it may comprise one or more selected from the group consisting of polyethylene glycol, monomethoxypolyethylene glycol, and combinations thereof.

The hydrophobic block is a polymer having biodegradability and may be a polymer of alpha (a)-hydroxy acid-derived monomer, and concretely, it may comprise one or more selected from the group consisting of polylactide, polyglycolide, poly(lactic-glycolide), polymandelic acid, polycaprolactone, polydioxan-2-one, polyamino acid, polyorthoester, polyanhydride, polycarbonate and combinations thereof, and more concretely, it may comprise one or more selected from the group consisting of polylactide, polyglycolide, poly(lactic-glycolide) and combinations thereof.

In an embodiment, the C₁ to C₅ alcohol may be methanol, ethanol, isopropanol or a mixture thereof. The alcohol may be an aqueous solution form dissolved in water, or a mixture with organic solvent such as acetone, tetrahydrofuran, acetic acid, acetonitrile, dioxane or a combination thereof.

In an embodiment, the mixing of effective ingredient, amphiphilic block copolymer and alcohol in step (1) can be conducted at a temperature of 40 °C to 70 °C for 1 hour to 6 hours.

In an embodiment, the removal of C₁ to C₅ alcohol in step (2) can be conducted at a temperature higher than 30 °C, and more concretely, it can be conducted at a temperature higher than 30 °C and lower than 50 °C for a time less than 2 hours.

More concretely, the removal of alcohol can be conducted at a temperature higher than 30 °C to 45 °C, or 32 °C to 42 °C, for 30 minutes to 100 minutes, 40 minutes to 80 minutes, or 50 minutes to 70 minutes. If the temperature for removing alcohol is too high, related substances may increase, and if the temperature is too low, it may be difficult to remove the alcohol sufficiently.

The amount of the C₁ to C₅ alcohol in the resulting mixture after the removal of alcohol (i.e., the mixture before micellization) is less than 2.5 w/v% (i.e., 0 to less than 2.5 w/v%), and more concretely, it may be 2.0 w/v% or less, 1.5 w/v% or less, 1.0 w/v% or less, 0.8 w/v% or less, 0.6 w/v% or less, or 0.5w/v% or less. If the alcohol remains in the mixture before micellization in an amount of 2.5 w/v% or more, the phenomenon of drug or polymer precipitation may be generated.

The aqueous medium used in the step of forming micelle may be, for example, selected from the group consisting of conventional water, distilled water, distilled water for injection, physiological saline, 5% glucose, buffer, and combinations thereof, but it is not limited thereto.

In an embodiment, the formation of micelle can be conducted at a temperature lower than 35 °C, and more concretely, it can be conducted at a temperature higher than 10 °C and lower than 35 °C.

More concretely, the micelle can be formed at 15°C to 32°C, 20°C to 30°C, or 25°C to 28°C. If the temperature when forming micelle is too high, related substances may increase or drug precipitation may happen, and if the temperature is too low, the mixture of polymer and drug is not dissolved sufficiently, and thus the micelle cannot be formed well or it may take too long time for the formation. In addition, the micellization can be conducted for less than 5 hours, 30 minutes to 4 hours, 60 minutes to 4 hours, or 90 minutes to 3 hours and 30 minutes.

In an embodiment, the step of forming micelle can comprise a procedure of filtering the formed micelle.

In an embodiment, the filtering can be conducted by using 0.1 to 0.8 µm, 0.1 to 0.6 µm, or 0.2 to 0.5 µm filter at 0 °C to 25 °C, 0 °C to 20 °C, or 0 °C to 15 °C. The filtering, if necessary, may be conducted by using different filters at different temperature conditions subsequently, or it may be repeated several times additionally according to each step.

In an embodiment, if the formed micelle (or formed and filtered micelle) is freeze-dried, the formed micelle can be maintained at 0 °C to 15 °C before the freeze-drying is conducted, and it can be freeze-dried within 3 hours. More concretely, the formed micelle can be maintained at 0 °C to 12 °C, or 0 °C to 10 °C before the freeze-drying is conducted, and can be applied to the freeze-drying step within 10 minutes to 3 hours, within 30 minutes to 3 hours, or within 1 hour to 3 hours.

In other embodiment, the formed micelle can be applied to the freeze-drying step directly, without storage procedure. "Maintaining" can be used for the same meaning as storing.

In an embodiment, the freeze-drying can be conducted in the presence of freeze-drying aid (also referred to as freeze-drying agent). The freeze-drying aid may be selected from the group consisting of sugar, sugar alcohol and mixtures thereof. The sugar may be one or more selected from the group consisting of lactose, maltose, sucrose and trehalose, and the sugar alcohol may be one or more selected from the group consisting of mannitol, sorbitol, maltitol, xylitol and lactitol. The freeze-drying aid is added so that the freeze-dried composition can maintain a cake form. In addition, after the freeze-drying of the polymer nanoparticle composition, the freeze-drying aid helps uniform dissolution in short time during the procedure of reconstitution. In an embodiment, the amount of the freeze-drying aid is 1 to 90 % by weight, and more concretely 10 to 60 % by weight, based on 100 % by weight of total dry weight of the freeze-dried composition.

In an embodiment, the effective ingredient can be poorly water-soluble drug.

In an embodiment, the poorly water-soluble drug can be selected from drugs having solubility in water (25 °C) of 100 mg/mL or less, and it also can be selected from antineoplastic agents, antifungal agents, immunosuppressants, analgesics, anti-inflammatory agents, antiviral agents, anxiolytic sedatives, contrasting agents, corticosteroids, diagnostic agents, diagnostic imaging agents, diuretics, prostaglandins, radiopharmaceuticals, sex hormones including steroids and combinations thereof, but it is not limited thereto.

In an embodiment, the poorly water-soluble drug can be selected from anticancer drugs, and concretely, it can be taxane anticancer drug. For example, the taxane anticancer drug can be one or more selected from the group consisting of paclitaxel, docetaxel, 7-epipaclitaxel, t-acetylpaclitaxel, 10-desacetylpaclitaxel, 10-desacetyl-7-epipaclitaxel, 7-xylosylpaclitaxel, 10-desacetyl-7-glutarylpaclitaxel, 7-N,N-dimethylglycylpaclitaxel, 7-L-alanylpaclitaxel and cabazitaxel, and more concretely, it can be paclitaxel, docetaxel, or a combination thereof.

Another aspect of the present invention provides nanoparticle comprising effective ingredient; amphiphilic block copolymer with a number average molecular weight of less than 3,800; and C₁ to C₅ alcohol; wherein the amount of the C₁ to C₅ alcohol is 0 to 2.5 w/v %.

The above nanoparticle can be that prepared by the preparation method according to an embodiment of the present invention.

In case of freeze-dried product, the nanoparticle according to an embodiment may have a reconstitution time in aqueous solution of 5 minutes or less, 4 minutes or less, or 3 minutes or less. The reconstitution time may be measured by a conventionally known method, and for example, it may be measured as a time taken to agitate the freeze-dried product nanoparticle in aqueous solution at a room temperature with 400 to 600 rpm until the solution becomes transparent.

After being formulated as a solution, the nanoparticle according to an embodiment may have a precipitation time of drug of 6 hours or more, 8 hours or more, 10 hours or more, or 12 hours or more. The precipitation time may be measured by a conventionally known method, and for example, when the nanoparticles are dissolved in aqueous solution to prepare a transparent solution and then the solution is kept at room temperature, it may be measured as a time taken until the solution becomes cloudy or precipitation is observed.

Examples are provided below in order to facilitate understanding of the present invention. However, the following examples are only to illustrate the present invention, and the scope of the present invention is not limited thereby in any manner.

### [EXAMPLES]

### Examples 1 to 3 and Comparative Example 1: Preparation of polymeric micelle containing docetaxel according to ethanol removal temperature

19 g of monomethoxypolyethylene glycol-(poly-D,L-lactic acid) copolymer (mPEG-PDLLA) and 1 g of docetaxel were weighed, and 4 ml of ethanol (EtOH) was added thereto and the mixture was agitated at 60 °C until the ingredients were completely dissolved and the solution became transparent. Then, by using a rotary evaporator equipped with a round bottom flask, ethanol was removed by distillation under reduced pressure according to the temperature and time conditions shown in the following Table 1. Then, the temperature was lowered to 28 °C and 230 ml of distilled water at room temperature was added thereto, and the reaction was conducted until the solution became transparent in blue color, to form polymeric micelle within 3 hours.

In case of preparation as a freeze-dried product, to the polymeric micelle formed as above, 5 g of mannitol as a freeze-drying agent was added directly and dissolved completely, and filtered by using a filter with 200 nm pore size, and then freeze-dried to prepare a docetaxel-containing polymeric micelle composition in powder form.

**[Table 1]**

| | **Number average molecular weight of mPEG-PDLLA** | **EtOH removal condition (Vacuum condition)** | | **Micellization condition** | | **Residual EtOH amount after EtOH removal (w/v%)** |
|---|---|---|---|---|---|---|
| | | **Temperature** | **Time** | **Temperature** | **Time** | |
| Comparative Example 1 | 3,550 | 30 °C | 2 hr | 28 °C | 2 hr | 0.95% |
| Example 1 | 3,550 | 35 °C | 1 hr | 28 °C | 2 hr | 0.50% |
| Example 2 | 3,550 | 40 °C | 1 hr | 28 °C | 2 hr | 0.48% |
| Example 3 | 3,360 | 40 °C | 1 hr | 28 °C | 2 hr | 0.49% |

### Example 4 and Comparative Examples 2 and 3: Preparation of polymeric micelle containing docetaxel according to micellization condition

The same procedure as the above Examples 1 to 3 and Comparative Example 1 was conducted, excepting that ethanol was removed by distillation under reduced pressure according to the conditions shown in the following Table 2. After the ethanol removal procedure, the residual ethanol amount was about 0.50 w/v%. Then, the temperature was lowered to the micellization temperature shown in the following Table 2, and 230 ml of distilled water at room temperature was added thereto, and the reaction was conducted for a predetermined time to form polymeric micelle.

**[Table 2]**

| | **Number average molecular weight of mPEG-PDLLA** | **EtOH removal condition** | | **Micellization condition** | |
|---|---|---|---|---|---|
| | | **Temperature** | **Time** | **Temperature** | **Time** |
| Comparative Example 2 | 3,550 | 35 °C | 1 hr | 35 °C | 2 hr |
| Comparative Example 3 | 3,360 | 40 °C | 1 hr | 35 °C | 1 hr |
| Example 4 | 3,460 | 40 °C | 1 hr | 28 °C | 3 hr |

### Example 5 and Comparative Example 4: Preparation of polymeric micelle containing docetaxel according to storage condition

The same procedure as the above Example 1 was conducted, excepting that the filtered micelle was stored under the conditions shown in the following Table 3, and 5 g of mannitol as a freeze-drying agent was added thereto and dissolved completely, and filtered by using a filter with 200 nm pore size, and then freeze-dried to prepare a docetaxel-containing polymeric micelle composition in powder form.

**[Table 3]**

| | Example 5 | Comparative Example 4 |
|---|---|---|
| Preparation condition | Freezing (< -70 °C) immediately after micellization at 28 °C | Freezing after keeping at room temperature for more than 3 hours after micellization at 28 °C |

### Experimental Example 1: Test for comparing precipitation time

To 2 g of polymeric micelle containing docetaxel prepared in each of Examples 1 to 3 and 5 and Comparative Examples 1, 3 and 4, 38 mL of physiological saline injection solution was added and dissolved by using IKA VIBRAX shaker, and then while keeping the solution, precipitation of docetaxel was observed at every 30 minutes. The results are shown in the following Table 4.

**[Table 4]**

| **Sample** | **Precipitation time** |
|---|---|
| Example 1 | 12 hours or more |
| Example 2 | 12 hours or more |
| Example 3 | within 10 hours |
| Example 5 | within 10 hours |
| Comparative Example 1 | within 7 hours |
| Comparative Example 3 | Impossible preparation due to the precipitation generated within 20 minutes |
| Comparative Example 4 | within 2 hours |

As can be seen from the above Table 4, in case of high residual amount of ethanol (Comparative Example 1), the precipitation time was remarkably reduced. In addition, in case of too high micellization temperature (Comparative Example 3), the preparation was impossible because the drug was precipitated during the micellization. Furthermore, in case of keeping at room temperature for a long time after the micellization (Comparative Example 4), the precipitation time was remarkably reduced.

### Experimental Example 2: Test for comparing related substance amount

The test solution was prepared by filtering the formulation solution of polymeric micelle containing docetaxel prepared in each of Examples 1 to 4 and Comparative Example 2 with 45 µm membrane filter paper and mixing 1 mL of the filtered solution with 4 mL of HPLC mobile phase solution. The prepared test solution was analyzed with HPLC under the following conditions to compare the amount of related substance. The amount of related substance (%) is shown in the following Table 5.

- HPLC conditions:
Column: Stainless column with 250 mm length and 4.6 mm inner diameter having silica gel particles with 5 µm particle diameter and 100 Å pore diameter, coated with C18, or a similar one thereto
Mobile phase: acetonitrile/methanol/water mixture solution (26/32/42, v/v/v) Flow rate: 1.5 ml/min
Detector: UV absorption spectrometer (Measured wavelength: 232 nm)

**[Table 5]**

| **Sample** | Impurity C in formulation solution |
|---|---|
| Example 1 | 0.14 % |
| Example 2 | 0.19 % |
| Example 3 | 0.14 % |
| Example 4 | 0.15 % |
| Comparative Example 2 | 0.35 % |

As can be seen from the above Table 5, in case of too high micellization temperature (Comparative Example 2), the amount of related substance in the formulation solution was increased.

### Experimental Example 3: Test for comparing reconstitution time

To 2 g of polymeric micelle containing docetaxel prepared in each of Examples 1 to 3, Comparative Example 1, Comparative Example 5 with different molecular weight of polymer, 38 mL of physiological saline injection solution was added, and then while dissolving the freeze-dried cake by using IKA VIBRAX shaker at shaking speed of 500 rpm, the time taken for complete dissolution (reconstitution time) was measured. The results are shown in the following Table 6.

**[Table 6]**

| **Sample** | **Number average molecular weight of mPEG-PDLLA** | **EtOH removal condition** | | **Micellization condition** | | **Reconstitution time** (min) |
|---|---|---|---|---|---|---|
| | | **Temperature** | **Time** | **Temperature** | **Time** | |
| Example 1 | 3550 | 35 °C | 1 hr | 28 °C | 2 hr | 4.5 |
| Example 2 | 3550 | 40 °C | 1 hr | 28 °C | 2 hr | 4.3 |
| Example 3 | 3360 | 40 °C | 1 hr | 28 °C | 2 hr | 2.9 |
| Comparative Example 1 | 3550 | 30 °C | 2 hr | 28 °C | 2 hr | 4.7 |
| Comparative Example 5 | 3800 | 35 °C | 1 hr | 35 °C | 2 hr | 7.3 |

## Claims

1. A method for preparing nanoparticle comprising:
(1) a step of mixing effective ingredient, amphiphilic block copolymer with a number average molecular weight of less than 3,800, and C₁ to C₅ alcohol;
(2) a step of removing C₁ to C₅ alcohol from the resulting mixture of step (1) to lower the amount of C₁ to C₅ alcohol in the mixture to less than 2.5 w/v %; and
(3) a step of adding aqueous medium to the resulting mixture of step (2) to form micelle.

2. The method for preparing nanoparticle of claim 1, wherein the removal of C₁ to C₅ alcohol is conducted at a temperature higher than 30 °C.

3. The method for preparing nanoparticle of claim 1, wherein the formation of micelle is conducted at a temperature lower than 35 °C.

4. The method for preparing nanoparticle of claim 1, wherein the step of forming micelle comprises a procedure of filtering the formed micelle.

5. The method for preparing nanoparticle of claim 1, further comprising a step of freeze-drying the micelle formed in step (3).

6. The method for preparing nanoparticle of claim 5, wherein the formed micelle is maintained at 0 °C to 15 °C before the freeze-drying is conducted.

7. The method for preparing nanoparticle of claim 5, wherein the formed micelle is freeze-dried within 3 hours.

8. The method for preparing nanoparticle of claim 1, wherein the amphiphilic block copolymer comprises hydrophilic block (A) selected from the group consisting of polyethylene glycol or derivative thereof, polyvinyl pyrrolidone, polyvinyl alcohol, polyacrylamide, and combinations thereof; and hydrophobic block (B) selected from the group consisting of polylactide, polyglycolide, poly(lactic-glycolide), polymandelic acid, polycaprolactone, polydioxan-2-one, polyamino acid, polyorthoester, polyanhydride, polycarbonate and combinations thereof.

9. The method for preparing nanoparticle of any one of claims 1 to 8, wherein the effective ingredient is poorly water-soluble drug.

10. Nanoparticle comprising effective ingredient; amphiphilic block copolymer with a number average molecular weight of less than 3,800; and C₁ to C₅ alcohol; wherein the amount of the C₁ to C₅ alcohol is 0 to 2.5 w/v %.
